(19)

Europäisches Patentamt
European Patent Office
Office européen des brevets

(11) **EP 3 085 345 A2**

(12) # EUROPEAN PATENT APPLICATION
published in accordance with Art. 153(4) EPC

(43) Date of publication:
**26.10.2016  Bulletin 2016/43**

(21) Application number: **14870709.4**

(22) Date of filing: **17.12.2014**

(51) Int Cl.:
***A61F 13/53*** (2006.01)

(86) International application number:
**PCT/IB2014/067045**

(87) International publication number:
**WO 2015/092721 (25.06.2015 Gazette 2015/25)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **18.12.2013  MX 2013015096**

(71) Applicant: **Grupo P.I. Mabe, S.A. de C.V.**
**72230 Puebla (MX)**

(72) Inventors:
• **SALCEDO AGUALLO, José**
  **Puebla 72230 (MX)**

• **CANALES ESPINOSA DE LOS MONTEROS, Carlos**
  **Puebla 72230 (MX)**
• **SÁNCHEZ FERNÁNDEZ, Lucía del Carmen**
  **Puebla 72230 (MX)**
• **MARTINEZ ZAMORA, Olimpia**
  **Puebla 72230 (MX)**

(74) Representative: **Gil-Vega, Victor**
  **Corazón de Maria, 6**
  **28002 Madrid (ES)**

(54) **IMPROVEMENTS IN THE DESIGN OF AN ABSORBENT MATRIX FOR A NAPPY FOR NEWBORN BABIES**

(57)    An absorbent core for implementation in a new-born baby diaper that has a front section, a back section and a crotch section so that the crotch section has a maximum width of 5 cm to allow the baby's legs to form an angle "α" at a maximum of 30° with respect to the vertical axis; in addition, said core comprises a base weight between 700 and 900 g/m2; a density that varies in a range between 0.16 and 0.24 g/cm3 and a ratio of absorbent to superabsorbent material from 50/50 to 70/30.

**FIG.1**

EP 3 085 345 A2

**Description**

**FIELD OF THE INVENTION**

The embodiments illustrated in the present invention generally refer to diapers for newborn babies.
More concretely, the invention proposes an ergonomically designed diaper that has an absorbent core that is at the most 5 cm thick wide between the legs.

**BACKGROUND OF THE INVENTION**

Currently, multiple varieties of diapers in various sizes and shapes are used, generally for babies and toddlers; however, there are also diapers for adults and even pets on the market today. In any case, the current market has been more concerned with producing diapers for older children, without developing solutions for the growth of a newborn baby's hips during the first few months. Often, newborn diapers currently available on the market now measure anywhere from 6 to 8.5 cm between the legs, forcing the babies to open their legs. In addition, if the absorbent core does not adapt to a newborn baby's dimensions, leakage can occur from the openings between the baby's legs and the diaper.
Currently, there are various proposals for newborn diapers, for example, the U.S. patent US 5,295,986 which describes a diaper for newborn babies that is adjustable, with a lengthening flap that can be fastened to the front section of the diaper, and which can also be adjusted around the belt with fasteners that can be attached to each other or to the front of the diaper when putting it on the baby, loosening or tightening the belt as needed.
As can be appreciated in the patent US 5,295,986 A, it is aimed at a product with certain dimensions which can be adjusted as the baby grows, in contrast to the present invention that features a core adjustable to the size of a newborn baby and that is comfortable for the baby, preventing diaper rash.
The international patent application WO2003009792 A1 describes a disposable diaper for newborn babies aimed at protecting the umbilical stump that remains attached to the newborn until it dries and falls off. To this end, there is a depression in the part of the diaper that covers the belly button as to not irritate the area. As can be seen in the international patent application WO2003009792 A1, even though it refers to a disposable diaper for newborn babies, it is not directed at providing the adjustability and comfort in the crotch area as is the present invention.
None of the previous cited documents describes a diaper for newborns that has an absorbent core in the specific dimensions that allows the baby to keep its legs at less than a 30° angle to its sides, preventing diaper rash in the hip area. Therefore, there is a need for a newborn diaper that overcomes all the inconveniences of previously developed diapers.

**OBJECTIVES**

The main objective of the present invention is: to provide a newborn diaper that comprises an ergonomically designed absorbent core that is no more than 5 cm wide between the legs.
Another objective of the present invention is to provide a diaper for newborn babies that comprises an absorbent core with a width between the legs that allows the baby to keep its legs at an angle $\alpha$ from the horizontal axis, less than 30°.
Another objective of the present invention is to provide a diaper for newborn babies, which has an absorbent core with a User Comfort Index (UCI) greater than or equal to 0.5.

**BRIEF DESCRIPTION OF THE DRAWINGS AND FIGURES**

The aforementioned aspects and many of the advantages associated with this invention will be more evident upon consideration of the following detailed description and viewed in conjunction with their accompanying drawings, wherein:

Figure 1 is a plan view of a baby diaper, size 0, typically found in the market, illustrating the type of absorbent core used.

Figure 2 is plan view of the absorbent core of the present invention, illustrating the type of absorbent core used.

Figure 3 is a frontal view of the angle formed between the baby's legs and the vertical axis.

**DETAILED DESCRIPTION OF THE INVENTION**

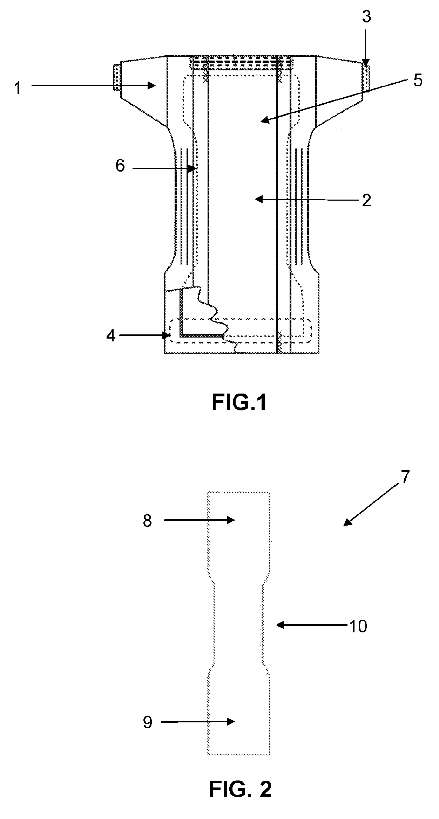

Figure 1 shows a plan view of a disposable diaper. As can be seen in the figure, disposable diapers are essentially made of: an outer permeable layer (2), an inner impermeable layer (4) and an absorbent core (5) containing fibers of an absorbent material and particulates of a superabsorbent material, placed between the outer and inner layers, external anti-leak barriers (6), fastening system (3), waist and leg elastic means (1) and bonding means; the article can additionally include one or more elastic areas.

The absorbent core (5) used in the disposable diapers can have a generally rectangular shape, or an "I" or "T" shape, or any other shape suitable to its purpose.

Figure 2 shows a plan view of the preferred shape of the absorbent core (7) of the present invention, which has a front section (8), a back section (9) and a crotch section (10), which is placed between the when the article is in use. The crotch section of the core has a maximum width of 5 cm for the purposes of the present invention.

To reduce the width of the core between the legs, a complete redesign of the core is necessary, given that this area is generally where bodily fluids are deposited when the product is in use, it is required that the core has an adequate balance of absorbent material and superabsorbent material for achieving the required absorption and retention of the bodily fluids, as well as an adequate base weight and density, such that these factors, combined with a maximum width of 5 cm between the legs, result in an absorbent core that is able to adequately receive, distribute and store the fluids, with the additional advantage of not forcing the baby to open its legs, allowing the baby instead to lie in a more natural position and therefore be more comfortable.

In consideration of the foregoing, the absorbent core of the invention has a base weight in the crotch section between 700 and 900 g/m$^2$, a density that varies between 0.16 and 0.24 g/cm$^3$ and a ratio of absorbent to superabsorbent material between 50/50 and 70/30; allowing the crotch section of the absorbent core of the invention to have a User Comfort Index (UCI) greater than or equal to 0.5. This UCI is a measurement of the relationship between density to base weight and the width of an absorbent core between the legs, and is calculated using the following formula:

$$UCI = (densidad/peso\ base)/ancho\ de\ entrepierna$$

The greater the width between the legs, the lesser the User Comfort Index.

Moreover, it is convenient to use a reception distribution layer placed over the absorbent core, which helps collect and distribute the liquid and to keep the diaper surface dry; a non-woven fabric made of polypropylene, polyester, rayon, cotton, PLA, PHA, bi-component fibers, or blends thereof, or any other type of fiber suitable for this purpose can be used as a reception distribution layer.

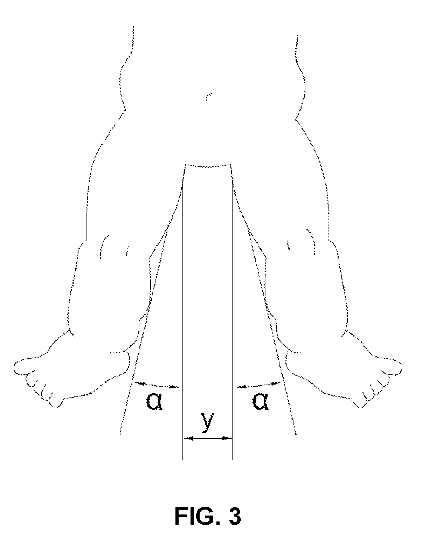

Babies weighing between 2.5 and 5 Kg that fit disposable diapers in sizes 0 and 1 have an average width between the legs (perinea) of 2.8 cm; this width is labeled with the letter "y" in Figure 3. Disposable diapers in sizes 0 and 1 that are available on the market comprise an average width between the legs from 6 to 8.5 cm, so that when putting these diapers on the baby, it is forced to lie with its legs open, at an angle "α" to the vertical axis. The greater the width of the crotch section of the diaper, the greater the angle α will be, meaning that the baby will keep its legs open, thereby adopting an unnatural position.

Table 1 shows the result of a series of trials where the width of the crotch section of the customary size 0 and size 1 diapers was measured, and was determined the angle α of opening of the baby legs during the use of these diapers.

| TABLE 1 | | |
|---|---|---|
| Sample A | Width between the legs (cm) | Angle "α" |
| Size 0 | 6.1 | 33 |
| Size 1 | 7.5 | 42.77 |
| Sample B | | |
| Size 0 | 7 | 39.58 |
| Size 1 | 7 | 39.58 |
| Sample C | | |
| Size 0 | 7.1 | 40.29 |
| Size 1 | 8.35 | 47.53 |
| Sample D | | |
| Size 1 | 7.35 | 42.77 |

(continued)

| Sample E | | |
|---|---|---|
| Size 1 | 7.5 | 41.84 |

With respect to the present invention, the proposed absorbent core has a width of 5 cm or less between the legs, whereby the baby only has to open its legs at an angle "$\alpha$" of 23.41°. To this end, the purpose of the present invention is that the angle "$\alpha$" does not exceed 30°, allowing the baby to assume the most natural position possible, this objective is achieved, designing the absorbent core so that its maximum width between the legs is 5 cm.

An embodiment of the invention is based on the fact that the absorbent core of the diaper for newborns can come in any form as long as its maximum width in the crotch section is 5 cm.

The absorbent core of the invention can be utilized with any type of disposable diaper.

Even though the invention has described in terms of its preferred embodiments, it should be evident to any field expert that any type of change or modification thereof falls within the scope of same.

**Claims**

1. An absorbent core to be used in a diaper for newborn babies that has a front section, a back section, and a crotch section, characterized because the crotch section has:

   A maximum width of 5 cm that allows the baby's legs to form an angle "$\alpha$" no wider than 30° with respect to the vertical axis;
   A base weight between 700 and 900 $g/m^2$;
   A density between 0.16 and 0.24 $g/cm^3$; and
   A ratio of absorbent to superabsorbent materials between 50/50 and 70/30.

2. An absorbent core of a newborn diaper in accordance with claim 1, characterized because the angle "$\alpha$" is 23.41°.

3. An absorbent core of a newborn diaper in accordance with claim 1, characterized because the absorbent core has a User Comfort Index (UCI) equal or greater than 0.5.

4. An absorbent core of a newborn diaper in accordance with claim 1, characterized because the absorbent core has in its upper face, a reception distribution layer.

5. An absorbent core of a newborn diaper in accordance with claim 1, characterized because it is in a rectangular shape.

6. An absorbent core of a newborn diaper in accordance with claim 1, characterized because it has an "I" shape.

7. An absorbent core of a newborn diaper in accordance with claim 1, characterized because it has a "T" shape.

3

1

5

2

6

4

**FIG.1**

7

8

10

9

**FIG. 2**

**FIG. 3**

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5295986 A

- WO 2003009792 A1